# EUROPÄISCHE PATENTSCHRIFT

(11) **EP 1 093 779 B1**
(45) Veröffentlichungstag und Bekanntmachung des Hinweises auf die Patenterteilung: **01.12.2004**
(21) Anmeldenummer: 00121543.3
(22) Anmeldetag: 30.09.2000
(51) Int. Cl.: A61F 13/00, A61F 13/04, A61F 13/06, A61F 13/10

(54) **Bandage zur Entlastung der Muskulatur beim Muskelfaserriss**
Bandage for reliefing a torn muscle
Bandage pour décharger un muscle déchiré

(30) Priorität: 20.10.1999 DE 19950509
(43) Veröffentlichungstag der Anmeldung: 25.04.2001
(73) Patentinhaber: Beiersdorf Aktiengesellschaft, 20245 Hamburg (DE)
(72) Erfinder: Kandt, Olaf, 52134 Herzogenrath (DE)

(56) Entgegenhaltungen:
- EP-A- 0 842 648
- US-A- 3 888 244

## Beschreibung

Die Erfindung betrifft eine Bandage zur Entlastung der Muskulatur beim Muskelfaserriß insbesondere an den Extremitäten des menschlichen Körpers.

Bei Beanspruchungen des Muskelapparates des menschlichen Körpers kommt es insbesondere bei sportlichen Aktivitäten immer wieder zu Muskelzerrungen, Muskelüberdehnungen, Muskelfaserrissen und Muskelbündelrissen. Diese Indikationen gehen teilweise mit großen Schmerzen einher, gerade die Muskelfaserrisse, die bei akuter oder chronischer Überforderung eines Muskels entstehen. Als Symptome zeigen sich neben den bereits erwähnten Schmerzen wie Bewegungs-, Anspannungs-, Druck- und Dehnschmerz auch Hämatome.
Gerade im Breitensport und im Leistungssport sowie im alltäglichen Leben gibt es nur wenige Verletzungen, die in einer so großen Häufigkeit auftreten wie der Muskelfaserriß und das Distorsionstrauma beziehungsweise der Bänderriß am OSG.

Therapeutisch werden Muskelfaserrisse u. a. durch Ruhigstellen des Muskels für 24 bis 48 Stunden, Kälteanwendungen im Verletzungsbereich und Hochlagerung des betroffenen Glieds behandelt. Weiterhin kommen noch Druckverbände zum Einsatz.
Des weiteren sind Salbenverbände und Zinkleimverbände bekannt sowie der Ansatz der physikalischen Therapie, die allgemein die Anregung oder gezielte Behandlung gestörter physiologischer Funktionen mit physikalischen, naturgegebenen Mitteln umfaßt, wie zum Beispiel Wasser, Wärme und Kälte, Licht, Luft, statisch-mechanisch (Massage), mit dynamischen Kräften, Heilquellen oder Elektrizität.

Die Ausgestaltung des anzulegenden Druckverbandes richtet sich nach dem verletzten Körperteil, sei es der Ober- oder Unterschenkel, sei es der Ober- oder Unterarm.

Üblicherweise werden Kompressionsverbände angelegt, die unter Verwendung von nicht klebenden und selbstklebend ausgerüsteten Verbänden hergestellt werden. Die betroffene Extremität wird zirkulär unter Aussparung des Verletzungsbereiches mit den nicht klebenden Verbänden umgeben. Die Fixierung wird mit sich komährenartig überkreuzenden zirkulären Zügeln aus dem klebenden Material vorgenommen, wobei diese distal und proximal der verletzten Stelle angebracht werden, um somit eine Entlastung der geschädigten Muskulatur zu erzielen, wobei der eigentliche Verletzungsbereich stets frei bleibt. Dieser Bereich kann mit Salben in seiner Genesung unterstützt werden, wobei die Salbe ohne Wechsel des Gesamtverbandes erfolgen kann.
Nachdem der Verband angelegt worden ist, sollte aber - um die Bildung eines Fensterödems auszuschließen - diese ausgesparte Stelle ebenfalls verschlossen werden.

Aus der EP 842648 A1 ist eine Bandage bekannt, die zwingend am Knie angelegt werden soll. Die hier offenbarte Fertigbandage ist auf einer Seite selbstklebend beschichtet und dient zur Entlastung und Stabilisierung von Band- und Muskelstrukturen am Kniegelenk. Sie besteht aus einem im wesentlichen rechteckigen, in Querrichtung der Bandage mindestens teilweise unelastischen mittleren Teil A, an welchem in Längsrichtung gesehen nach oben und unten gerichtet jeweils zwei längliche Streifen B, C, D und E angeordnet sind.

Die geschilderte funktionelle Verbandtechnik, auch Taping genannt, ist insgesamt eine Behandlungsmethode zur Prophylaxe und Therapie von Verletzungen, Krankheiten und Veränderungen am Bewegungsapparat. Taping hat zum Ziel, die Kapsel-Band-Strukturen gezielt nachzubilden und dadurch eine selektive Unterstützung und Stabilisierung zu erreichen.

Der eigentliche Tapeverband wird dabei streifenweise aus vorzugsweise unelastischen selbstklebenden Bändern, sogenannten Zügeln, oder in Verbindung mit kurzzugelastischen selbstklebenden Bändern angelegt. Er schützt, stützt und entlastet gefährdete, geschädigte oder gestörte Anteile einer Funktionseinheit. Er erlaubt die selektive Belastung im schmerzfreien Bewegungsraum, verhindert aber extreme oder schmerzhafte Bewegungen.

Das Anlegen derartiger Verbände erfordert jedoch fachmännisches Können und Erfahrung und kann deshalb in aller Regel nicht von Laien ohne Taping-Erfahrung ausgeführt werden.

Dies trifft auch bei den bekannten Verbänden zur Therapierung von Muskelfaserrissen oder -zerrungen zu. Der Laie ist ohne weiteres nicht in der Lage, den ausführlich beschriebenen Verband ohne ärztliche oder fachmännische Hilfe anzulegen.

Weiterhin kommen als Mittel zur Behandlung Orthesen in Frage, wobei die Orthese rein medizinisch gesehen eher eine Versorgung darstellt, die normalerweise für diese Indikationen eine Unterversorgung darstellt.

Orthopädische Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine dreidimensionale Form aufweisen, um den anatomischen Gegebenheiten zu entsprechen, um form- und kraftschlüssig von extern auf den menschlichen Körper einwirken zu können.

Aufgabe der Erfindung ist es, eine Bandage zu entwickeln, die bei Muskelzerrungen, Muskelüberdehnungen, Muskelfaserrissen und Muskelbündelrissen erfolgreich eingesetzt werden kann, ohne dabei die oben erwähnten Mängel aufzuweisen. Insbesondere soll die Bandage problemlos anzulegen, einen hohen Tragekomfort bieten und einfach nachzujustieren sein, falls dies erforderlich ist Weiterhin soll die Bandage die verletzte Stelle entlasten.

Gelöst wird diese Aufgabe durch eine Bandage, wie sie im Hauptanspruch niedergelegt ist. Gegenstand der Unteransprüche sind dabei vorteilhafte Weiterbildungen der Bandage.

Demgemäß betrifft die Erfindung eine Bandage zur Entlastung der Muskulatur, umfassend einen im wesentlichen rechteckigen Zentralabschnitt, an dessen Querkanten vorzugsweise in den Eckbereichen insgesamt vier Zuggurte befestigt sind.
Der Zentralabschnitt wird gegenüber der verletzten Stelle im Muskelgewebe in der Extremität angeschmiegt. Die beiden proximalen Zuggurte werden in einer diagonalen Bewegung nach distal und die beiden distalen Zuggurte in einer diagonalen Bewegung nach proximal derartig geführt, daß sich die beiden proximalen Zuggurte proximal der verletzten Stelle, die beiden distalen Zuggurte distal der verletzten Stelle überkreuzen und jeweils ein proximaler und ein distaler Zügel sich seitlich der verletzten Stelle überkreuzen, so daß die verletzte Stelle ausgelassen wird, und wobei die Enden der Zuggurte auf der Bandage befestigt werden.

Es ergibt sich auf diese Art und Weise ein offener Abschnitt über der verletzten Stelle, so daß die Gurte ihre Wirkung direkt auf der Haut ausüben können, um somit eine effektive Entlastung der verletzten Stelle zu erreichen.

Der Zentralabschnitt der Bandage sollte aus einem festen, aber dehnfähigen Material gefertigt sein, vorzugsweise aus einem längselastischen Gewebe oder Gewirke, das gegebenenfalls auch eine geringe Querelastizität aufweisen kann, insbesondere auf Baumwollbasis. Die Längselastizität entspricht vorzugsweise derjenigen von sogenannten Kurzzugbinden, d.h. Binden mit einer Dehnungsfähigkeit von ungefähr 60 % bis 90 %.

In einer vorteilhaften Ausführungsform der Bandage vereinigen sich jeweils ein proximaler Zuggurt und ein distaler Zuggurt seitlich der verletzten Stelle zu einem einzigen Gurt, so daß sich zwei Y-förmige Zuggurte ergeben.

Die Gurte weisen hierzu vorzugsweise an ihren Enden Klettverschlußbänder auf und werden mittels Zurrösen auf sich selbst befestigt.

Durch diesen Y-förmigen Zuggurt kann man mit nur zwei Handgriffen die Bandage mit dem gewünschten Druck anlegen und somit die komplette Muskulatur entlasten.

Im V-Bereich besteht der Gurt vorteilhafterweise aus einem weichen, dünnen und kurzzugelastischen Material wie Gewebe oder Gewirke. Fallweise können sich auch elastische oder plastische Anteile in Längs- oder in Querrichtung vorteilhaft auf die Anwenderempfindung auswirken. Weiterhin können auch Vliesstoffe oder Schäume oder Papier eingesetzt werden, wenn diese eine ausreichende Festigkeit aufweisen. Für die Enden der Gurte werden insbesondere unelastische Materialien verwendet.

Vorzugsweise kann das Material aus Baumwolle bestehen, des weiteren eine Höchstzugkraft von nicht weniger als 50 N/cm und eine Höchstzugkraft-Dehnung von weniger als 20% aufweisen.

Den Unterschenkel sollten die Gurte nur semizirkulär umfassen, um eine Stauung auszuschließen.

Um einen hohen Zug ausüben zu können, können am Zentralabschnitt Zurrösen angebracht sein, durch die die Gurte geführt und anschließend auf sich selbst befestigt werden.
Durch die Zurrösen wird ermöglicht, daß die Gurte nicht zirkulär angelegt werden, was die Gefahr einer Stauung erheblich verringert.

Für die Oberschenkelvariante der erfindungsgemäßen Bandage kann man auf die Zurrösen verzichten, hier sind Gurte mit breiten Klettenden vorteilhaft.

Besonders gut läßt sich die Bandage an der betroffenen Extremität sichern, wenn am proximalen Ende des Zentralabschnitts ein Klettverschlußband und am distalen Ende des Zentralabschnitts ein Klettverschlußband angebracht sind. Ein Verrutschen ist somit ausgeschlossen. Die Verschlußbänder sollen vorzugsweise maximal ¾ die Extremität umfassen, um eine Stauung zu vermeiden.

Insbesondere für den Fall, daß die Bandage über der Tibia liegt, ist in den Zentralabschnitt ein vertikal verlaufender, weicher Abschnitt eingesetzt, um Druckstellen auf die Tibia zu vermeiden.

Um unterstützende Behandlungsmaßnahmen zu erleichtern und um der Ausbildung eines Fensterödems vorzubeugen, kann in einer weiteren vorteilhaften Ausführungsform der Bandage am Zentralabschnitt eine Verschlußlasche angebracht sein, die die verletzte Stelle reversibel überdeckt.
Die Verschlußlasche ist am Zentralabschnitt vorzugsweise angenäht, und zwar derartig, daß die Zuggurte beziehungsweise der Y-förmige Zuggurt zwischen Zentralabschnitt und Verschlußlasche durchgeführt werden kann, um das Spannen der Bandage nicht zu behindern.
Am einfachsten ist hierzu die Verschlußlasche nur jeweils am Randbereich mit dem Zentragabschnitt vemäht.

So ist es möglich, jederzeit an diese Stelle heranzukommen, ohne die Bandage ausziehen zu müssen. Die Bildung eines Fensterödems wird sicher vermieden.
Die Verschlußlasche sollte bevorzugt ebenfalls aus einem Material bestehen, welches relativ fest ist und einen kurzen Zug aufweist.

Dann kann die Verschlußlasche mit einem weichen Pad versehen sein, damit die verletzte Stelle zusätzlich komprimiert werden kann. Das Pad kann gleichzeitig als Salbenträger dienen.
Vorteilhafterweise wird das Pad auf der Verschlußlasche mittels eines Klettverschluß befestigt, um somit einen schnellen und einfachen Wechsel zu ermöglichen.
Des weiteren kann das Pad doppelwandig ausgeführt sein beziehungsweise eine aufgenähte Tasche aufweisen, um Kühlelemente in das Pad beziehungsweise in die Tasche einführen zu können, die eine Primärversorgung gewährleisten.
Ein geeignetes Material zur Bildung des Pads stellt zum Beispiel ein textilkaschierter Latexschaum dar, der entsprechend flexibel ist und sich an stark konturierte Körperregionen gut anlegen läßt. U.a. ist solch Latexschaum bei der Firma Beiersdorf unter der Bezeichnung "Leukotape Foam ®" erhältlich.

Des weiteren sind in einer bevorzugten Variante an der Bandage seitliche Stabilisatoren angebracht, zum Beispiel aus dünnen Kunststoffrollen.

Zusammenfassend kann festgehalten werden, daß sich die erfindungsgemäße Bandage bei Muskelzerrungen, Muskelüberdehnungen, Muskelfaserrissen und Muskelbündelrissen hervorragend ohne Kontraindikation anwenden läßt.

Eine weitere vorteilhafte Verwendung der Bandage erfolgt in Verbindung mit einem Thromboseprophylaxe-Strumpf, wie sie beispielsweise von der Firma Beiersdorf unter dem Namen "Comprinet ® S" oder "Comprinet ®" angeboten werden.

Das Prinzip der erfindungsgemäßen Bandage beruht auf den Wirkungsweisen der sehr erfolgreich bei den erwähnten Indikationen angewendeten Tape-Verband-Technik.

Durch das Aneinanderbringen der verletzten Muskelfasem wird eine Schmerzentlastung im verletzten Gebiet erreicht. Die Schmerzentlastung ist aber nicht permanent, sondern erfolgt nur dann, wenn bei Kontraktion der Muskulatur die verletzten Muskelfasern belastet werden.

Immer, wenn es zu einer Belastung im verletzten Gebiet des Muskels kommt, erfährt der Patient eine Entlastung an der verletzten Stelle, d.h. eine deutliche Schmerzlinderung im verletzten Gebiet insbesondere beim Gehen.

Dies wiederum hat zur Folge, daß sich der Patient oder Sportler im alltäglichen Bewegungsablauf wie Gehen, Laufen oder Autofahren etc. durch die Schmerzentlastung physiologischer bewegen wird und er das Bein früher wieder normal benutzen wird.

Die erfindungsgemäße Bandage ist mit nur wenigen Handgriffen anzulegen. Des weiteren wird aufgrund der hohen Flexibilität mit nur wenigen Größen ein großes Patientenspektrum abgedeckt.

Ein weiterer für den Fachmann nicht erwarteter Vorteil der Bandage besteht darin, daß die Bandage beliebig angewendet werden kann, also gegebenenfalls um 180° gewendet oder um einen beliebigen Winkel um die Extremität gedreht werden, ohne Veränderungen an der Bandage vornehmen zu müssen.

Anhand der nachfolgend beschriebenen Figuren werden besonders vorteilhafte Ausführungsformen der Bandage näher erläutert, ohne damit die Erfindung unnötig einschränken zu wollen. Es zeigen
- Figur 1: die Bandage in der einfachsten Ausführungsform mit vier Zuggurten,
- Figur 2: die Bandage in einer vorteilhaften Ausführungsform mit den Y-förmigen Zuggurten,
- Figur 3: die Bandage in der vorteilhaften Ausführungsform mit den Y-för migen Zuggurten gemäß Figur 2, angelegt am rechten Oberschenkel eines Patienten, wobei sich die verletzte Stelle anterior im Muskelgewebe des Oberschenkels befindet,
- Figur 4: die Bandage in einer weiteren vorteilhaften Ausführungsform mit den Y-förmigen Zuggurten gemäß Figur 2 sowie mit einer Verschlußlasche, angelegt am rechten Oberschenkel eines Patienten, wobei sich die verletzte Stelle posterior im Muskelgewebe des Oberschenkels befindet,
- Figur 5: die Bandage in einer weiteren vorteilhaften Ausführungsform mit den Y-förmigen Zuggurten, die mittels Zurrösen verschlossen werden, sowie mit dem eingesetzten weichen Abschnitt, angelegt am rechten Unterschenkel eines Patienten, wobei sich die verletzte Stelle posterior im Muskelgewebe der Wade befindet, und
- Figur 6: die Bandage in einer vorteilhaften Ausführungsform, gegenüber der Bandage aus Figur 5 vereinfacht, angelegt am rechten Unterschenkel eines Patienten, wobei sich die verletzte Stelle posterior im Muskelgewebe der Wade befindet.

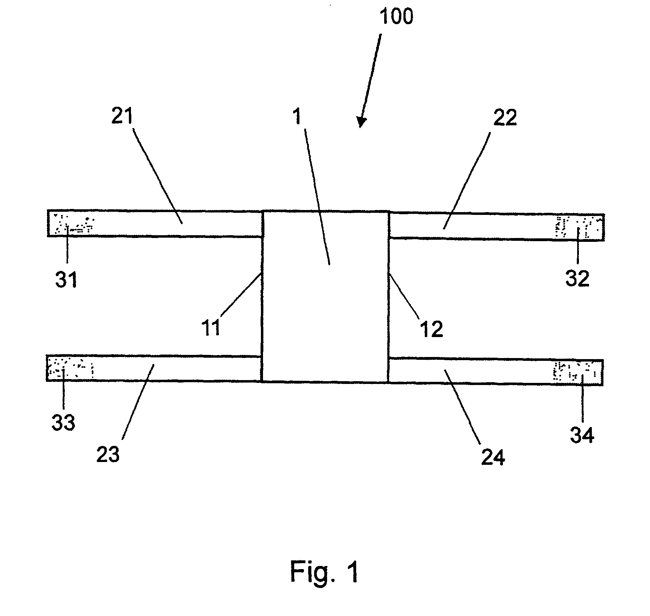

In der Figur 1 ist die Bandage 100 in der einfachsten Ausführungsform gezeigt mit vier Zuggurten 21, 22, 23, 24. Die Bandage 100 zur Entlastung der Muskulatur umfaßt einen im wesentlichen rechteckigen Zentralabschnitt 1, an dessen Querkanten 11, 12 in den Eckbereichen insgesamt vier Zuggurte 21, 22, 23, 24 befestigt sind.
Beim Anlegen der Bandage an einem der Arme oder einem der Beine wird der Zentralabschnitt 1 gegenüber der verletzten Stelle im Muskelgewebe in der Extremität angeschmiegt. Die beiden proximalen Zuggurte 21, 22 werden in einer diagonalen Bewegung nach distal und die beiden distalen Zuggurte 23, 24 in einer diagonalen Bewegung nach proximal derartig geführt, daß sich die beiden proximalen Zuggurte 21, 22 proximal der verletzten Stelle, die beiden distalen Zuggurte 23, 24 distal der verletzten Stelle überkreuzen und jeweils ein proximaler 21, 22 und ein distaler Zügel 23, 24 sich seitlich der verletzten Stelle überkreuzen, so daß die verletzte Stelle ausgelassen wird.
Die Enden 31, 32, 33, 34 der Zuggurte 21, 22, 23, 24 werden abschließend auf der Bandage 100 befestigt. Die Enden 31, 32, 33, 34 sind hierzu vorzugsweise mit einer Klettfläche ausgerüstet.

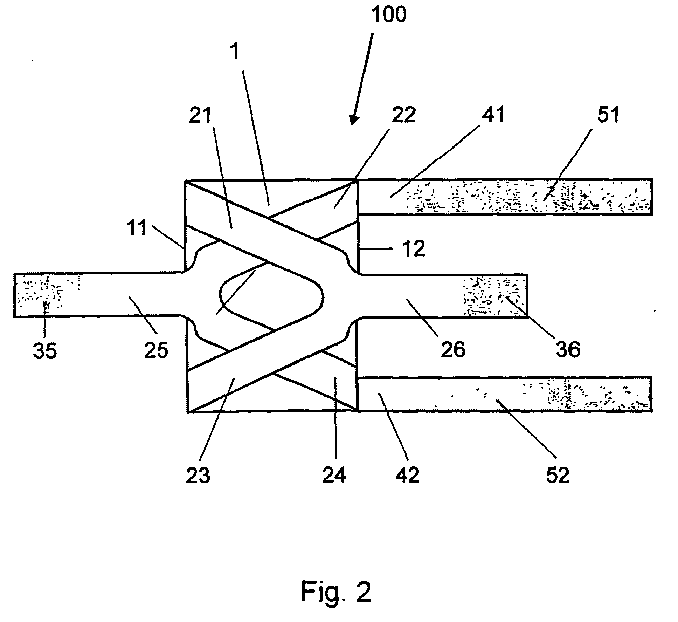

Die Figur 2 stellt die Bandage 100 in einer ersten vorteilhaften Ausführungsform dar. An den im wesentlichen rechteckigen Zentralabschnitt 1, an dessen Querkanten 11, 12 wieder insgesamt vier Zuggurte 21, 22, 23, 24 befestigt sind, sind zusätzlich am proximalen Ende des Zentralabschnitts 1 ein Klettverschlußband 41 und am distalen Ende des Zentralabschnitts 1 ein Klettverschlußband 42 angebracht.
Die Klettverschlußbänder 41, 42 tragen annähernd über die gesamte Länge Klettflächen 51, 52, um einen sicheren Sitz der Bandage an der Extremität zu gewährleisten.

Bei dieser besonders bevorzugten Variante der Bandage 100 vereinigen sich jeweils ein proximaler Zuggurt 21, 22 und ein distaler Zuggurt 23, 24 seitlich der verletzten Stelle zu einem einzigen Gurt, so daß sich zwei Y-förmige Zuggurte 25, 26 ergeben.
Die Zuggurte 25, 26 weisen an ihren Enden Klettverschlußbänder 35, 36 auf und werden mittels Zurrösen, die sich auf der Rückseite des Zentralabschnitts 1 befinden, auf sich selbst befestigt.

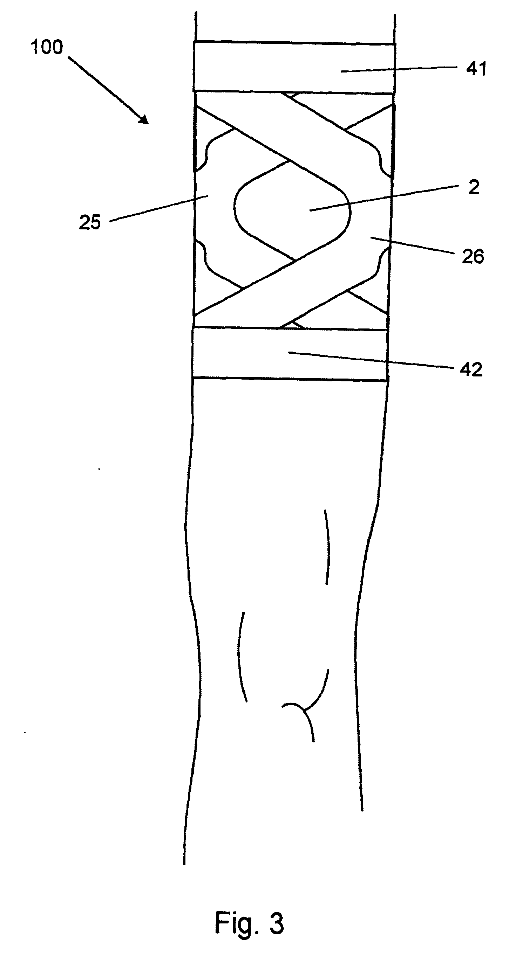

Die Figur 3 zeigt die Bandage 100 in der vorteilhaften Ausführungsform mit den Y-förmigen Zuggurten 25, 26 gemäß Figur 2, wobei die Bandage 100 am rechten Oberschenkel eines Patienten angelegt ist. Die verletzte Stelle 2 (Muskelfaserriß) befindet sich anterior im Muskelgewebe des Oberschenkels.

Mittels der gespannten Zuggurte 25, 26 werden die verletzten Muskelfasern aneinandergebracht, so daß eine Schmerzentlastung im verletzten Gebiet 2 erreicht wird. Die Schmerzentlastung ist aber nicht permanent, sondern erfolgt nur dann, wenn bei Kontraktion der Muskulatur die verletzten Muskelfasern belastet werden.

Durch das Umschließen der Bandage 100 durch die Klettverschlußbänder 41, 42 wird die Bandage 100 in ihrer Position gesichert.

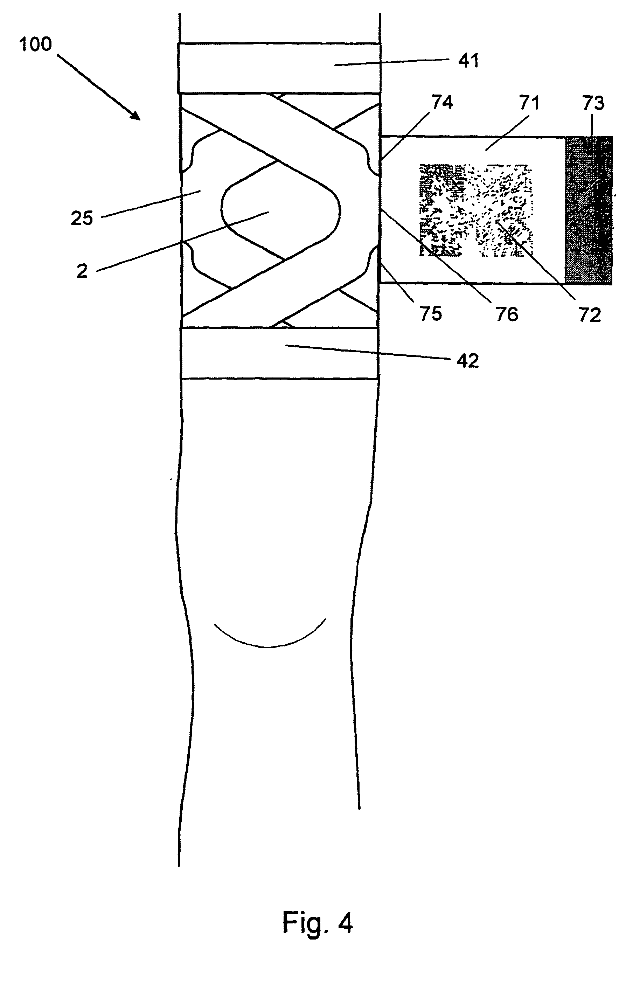

In der Figur 4 ist die Bandage 100 in einer weiteren vorteilhaften Ausführungsform mit den Y-förmigen Zuggurten 25, 26 gemäß Figur 2 sowie mit einer Verschlußlasche 71 offenbart.
Die Bandage 100 ist angelegt am rechten Oberschenkel eines Patienten, wobei sich die verletzte Stelle 2 posterior im Muskelgewebe des Oberschenkels befindet.
Die Verschlußlasche 71 ist am Zentralabschnitt 1 befestigt, wie üblich vorzugsweise angenäht, und zwar derartig, daß der Zuggurt 26 zwischen Zentralabschnitt 1 und Verschlußlasche 71 durchgeführt werden kann, um das Spannen des Zuggurtes 26 nicht zu behindern.
Die Naht, mit der die Verschlußlasche 71 am Zentralabschnitt 1 befestigt ist, ist mittig unterbrochen, so daß sich zwei Teilnähte 74 und 75 ergeben. Durch die daraus resultierende Lücke 76 wird der Zuggurt 26 geführt, also zwischen Zentralabschnitt 1 und Verschlußlasche 71.

Auf der Verschlußlasche 71 ist ein weiches Pad 72 vorhanden, um die Kompression auf die verletzte Stelle 2 zu erhöhen.

Zum Verschließen der Verschlußlasche 71 ist weiterhin eine Klettfläche 73 vorgesehen, die nach dem Umklappen der Verschlußlasche 71 auf der Bandage 100 selbst befestigt werden kann.

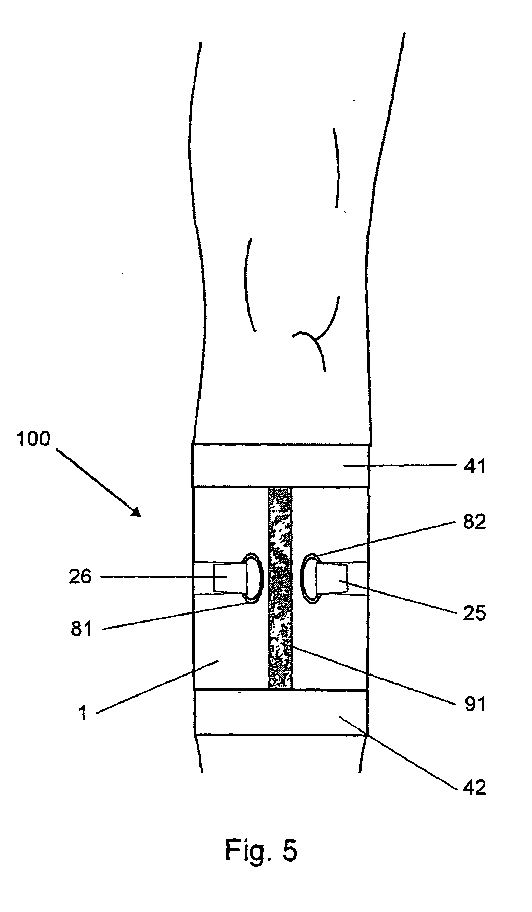

Mit der Figur 5 ist die Bandage 100 in einer weiteren vorteilhaften Ausführungsform gezeigt, und zwar weist die Bandage 100 die Y-förmigen Zuggurte 25, 26 auf, die mittels Zurrösen 81, 82 verschlossen werden, sowie den in den Zentralabschnitt 1 eingesetzten weichen Abschnitt 91. Die Bandage 100 ist am rechten Unterschenkel eines Patienten angelegt, wobei sich die verletzte Stelle posterior im Muskelgewebe der Wade befindet.

Die Zurrösen 81, 82 sind an der außen liegenden Seite des Zentralabschnitts 1 befestigt, und zwar vorzugsweise wieder angenäht.
Die Zuggurte 25, 26 werden durch die Ösen 81, 82 geführt, kräftig gespannt, so daß sich der erforderliche Druck aufbaut, und auf sich selbst mittels der Klettenden 35, 36 verschlossen.

Da die Bandage 100 über der Tibia liegt, ist in den Zentralabschnitt 1 der vertikal verlaufende, weiche Abschnitt 91 eingesetzt, um Druckstellen auf die Tibia zu vermeiden.

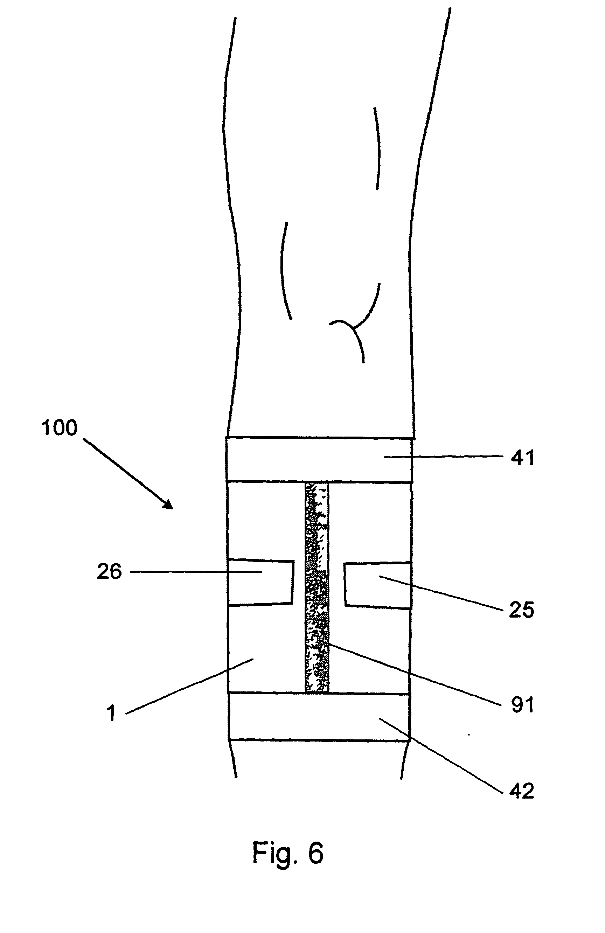

Figur 6 schließlich zeigt die Bandage 100 in einer weiteren vorteilhaften Ausführungsform. Die dargestellte Bandage 100 ist gegenüber der Bandage 100 aus Figur 5 vereinfacht, wobei die Bandage 100 wieder am rechten Unterschenkel eines Patienten angelegt ist und wobei sich die verletzte Stelle posterior im Muskelgewebe der Wade befindet.
Bei dieser Bandage 100 fehlen die Zurrösen. Der Druck wird aufgebaut, in dem die Zuggurte 25, 26 gespannt werden. Insbesondere bei leichteren Beeinträchtigungen des Muskelgewebes ist es ausreichend, die Zuggurte 25, 26 auf sich selbst mittels Klettflächen zu fixieren.

## Patentansprüche

1. Bandage (100) zur Entlastung der Muskulatur, umfassend einen im wesentlichen rechteckigen Zentralabschnitt (1), an dessen Querkanten (11, 12) insgesamt vier Zuggurte (21, 22, 23, 24) befestigt sind, wobei der Zentralabschnitt (1) gegenüber der verletzten Stelle im Muskelgewebe in der Extremität anschmiegbar ist, **dadurch gekennzeichnet, dass**
- die beiden proximalen Zuggurte (21, 22) in einer diagonalen Richtung nach distal und
- die beiden distalen Zuggurte (23, 24) in einer diagonalen Richtung nach proximal derartig führbar sind und
- jeweils ein proximaler Zuggurt (21, 22) und ein distaler Zuggurt (23, 24) sich seitlich der verletzten Stelle zu einem einzigen Gurt vereinigen, so dass sich zwei Y-förmige Zuggurte (25, 26) ergeben,
- so dass die verletzte Stelle ausgelassen wird, und
- wobei die Enden (31, 32, 33, 34) der Zuggurte (21, 22, 23, 24) auf der Bandage (100) zu befestigen sind.

2. Bandage gemäß Anspruch 1, **dadurch gekennzeichnet, dass** am proximalen Ende des Zentralabschnitts (1) ein Klettverschlußband (41) und am distalen Ende des Zentralabschnitts (1) ein Klettverschlußband (42) angebracht sind.

3. Bandage gemäß Anspruch 1 oder 2, **dadurch gekennzeichnet, dass** die Y-förmigen Zuggurte (25, 26) an ihren Enden Klettverschlußbänder (35, 36) aufweisen und mittels Zurrösen (81, 82) auf sich selbst befestigt werden.

4. Bandage gemäß einem der vorherigen Ansprüche, **dadurch gekennzeichnet, dass** in den Zentralabschnitt (1) ein vertikal verlaufender, weicher Abschnitt (91) eingesetzt ist.

5. Bandage gemäß einem der vorherigen Ansprüche, **dadurch gekennzeichnet, dass** am Zentralabschnitt (1) eine Verschlußlasche (71) angebracht ist, die die verletzte Stelle reversibel überdeckt.

6. Bandage gemäß einem der vorherigen Ansprüche, **dadurch gekennzeichnet, dass** an der Bandage (100) seitliche Stabilisatoren angebracht sind.

## Claims

1. Bandage (100) for relief of the musculature, comprising an essentially rectangular central portion (1) on whose transverse edges (11, 12) a total of four tensioning straps (21, 22, 23, 24) are secured, it being possible for the central portion (1) to be smoothed on opposite the injured site in the muscle tissue of the limb, **characterized in that**
- the two proximal tensioning straps (21, 22) are guided in a diagonal direction in the distal direction and
- the two distal tensioning straps (23, 24) can be guided in a diagonal direction in the proximal direction, and
- in each case a proximal tensioning strap (21, 22) and a distal tensioning strap (23, 24) come together laterally of the injured site to form a single strap, so that two Y-shaped tensioning straps (25, 26) are obtained,
- so that the injured site is left free,
- with the ends (31, 32, 33, 34) of the tensioning straps (21, 22, 23, 24) being secured on the bandage (100).

2. Bandage according to Claim 1, **characterized in that** a velcro fastener tape (41) is arranged at the proximal end of the central portion (1) and a velcro fastener tape (42) is arranged at the distal end of the central portion (1).

3. Bandage according to Claim 1 or 2, **characterized in that** the Y-shaped tensioning straps (25, 26) have velcro fastener tapes (35, 36) at their ends and are secured on themselves by means of fastener eyelets (81, 82).

4. Bandage according to one of the preceding claims, **characterized in that** a vertically extending, soft portion (91) is fitted in the central portion (1).

5. Bandage according to one of the preceding claims, **characterized in that** a fastening flap (71) is arranged on the central portion (1) and reversibly covers the injured site.

6. Bandage according to one of the preceding claims, **characterized in that** lateral stabilizers are arranged on the bandage (100).

## Revendications

1. Bandage (100) pour décharger la musculature, comprenant une portion centrale essentiellement rectangulaire (1) sur les arêtes transversales (11, 12) duquel sont fixées au total quatre sangles de traction (21, 22, 23, 24), la portion centrale (1) pouvant s'adapter à l'extrémité par rapport à la zone blessée dans le tissu musculaire, **caractérisé en ce que**
- les deux sangles de traction proximales (21, 22) peuvent être guidées dans une direction diagonale vers la direction distale et
- les deux sangles de traction distales (23, 24) peuvent être guidées dans une direction diagonale vers la direction proximale et
- à chaque fois une sangle de traction proximale (21, 22) et une sangle de traction distale (23, 24) se réunissent latéralement à la zone blessée pour former une sangle unique, de sorte que l'on obtienne deux sangles de traction en forme de Y (25, 26),
- de sorte que la zone blessée soit omise et
- les extrémités (31, 32, 33, 34) des sangles de traction (21, 22, 23, 24) devant être fixées au bandage (100).

2. Bandage selon la revendication 1, **caractérisé en ce que** l'on installe sur l'extrémité proximale de la portion centrale (1) une bande à fermeture de type velcro (41) et à l'extrémité distale de la portion centrale (1) une bande à fermeture de type velcro (42).

3. Bandage selon la revendication 1 ou 2, **caractérisé en ce que** les sangles de traction en forme de Y (25, 26) présentent à leurs extrémités des bandes à fermeture de type velcro (35, 36) et sont fixées à elles-mêmes au moyen de d'oeillets de fixation (81, 82).

4. Bandage selon l'une quelconque des revendications précédentes, **caractérisé en ce que** l'on insère dans la portion centrale (1) une portion molle (91) s'étendant verticalement.

5. Bandage selon l'une quelconque des revendications précédentes, **caractérisé en ce que** l'on installe sur la portion centrale (1) une patte de fermeture (71) qui recouvre la zone blessée de manière réversible.

6. Bandage selon l'une quelconque des revendications précédentes, **caractérisé en ce que** l'on installe sur le bandage (100) des stabilisateurs latéraux.
